# FASCICULE DE BREVET EUROPEEN

(11) **EP 2 285 390 B1**
(45) Date de publication et mention de la délivrance du brevet: **01.02.2012**
(21) Numéro de dépôt: 09750005.2
(22) Date de dépôt: 05.05.2009
(51) Int. Cl.: A61K 36/45, A61P 31/12, A61P 31/04, A61P 11/04, A61P 1/02

(54) **Procédé d'obtention d'un extrait de marc de canneberge pour son utilisation dans la prevention et le traitement d'affections telles que caries, gingivites ou maux de gorge**
Verfahren zur Gewinnung eines Extrakts aus Preiselbeertresten zur Prävention und Behandlung von Erkrankungen wie Karies, Zahnfleischentzündung oder Halsentzündung
Method for obtaining an extract of cranberry pulp residues for use in the prevention and treatment of conditions such as caries, gingivitis or sore throats.

(30) Priorité: 05.05.2008 FR 0802463
(43) Date de publication de la demande: 23.02.2011
(73) Titulaire: Tournay Biotechnologies, Sas, 24230 Lamothe Montravel (FR)
(72) Inventeur: TOURNAY, David, F-75017 Paris (FR); TOURNAY, Michaël, 33220 Sainte Foy La Grande (FR)
(74) Mandataire: Fourcade, Emmanuelle
(86) Numéro de dépôt international: PCT/FR2009/000523
(87) Numéro de publication internationale: WO 2009/141524

(56) Documents cités:
- WO-A-99/13889
- WO-A1-96/28135
- US-A- 5 474 774
- RAGHAVAN SIVAKUMAR ET AL: "Partitioning and inhibition of lipid oxidation in mechanically separated turkey by components of cranberry press cake" JOURNAL OF AGRICULTURAL AND FOOD CHEMISTRY, vol. 54, no. 17, août 2006 (2006-08), pages 6403-6408, XP002506150 ISSN: 0021-8561
- WOO A H ET AL: "ANTHOCYANIN RECOVERY FROM GRANBERRY PULP WASTES BY MEMBRANE TECHNOLOGY" JOURNAL OF FOOD SCIENCE, WILEY-BLACKWELL PUBLISHING, INC, US, vol. 45, no. 4, 1 janvier 1980 (1980-01-01), pages 875-879, XP000971709 ISSN: 0022-1147
- LAPORNIK B ET AL: "Comparison of extracts prepared from plant by-products using different solvents and extraction time" JOURNAL OF FOOD ENGINEERING, BARKING, ESSEX, GB, vol. 71, no. 2, 1 novembre 2005 (2005-11-01), pages 214-222, XP004923754 ISSN: 0260-8774
- BODET C CHARLES ET AL: "Inhibition of periodontopathogen-derived proteolytic enzymes by a high-molecular-weight fraction isolated from cranberry" JOURNAL OF ANTIMICROBIAL CHEMOTHERAPY 200604 GB, vol. 57, no. 4, avril 2006 (2006-04), pages 685-690, XP002506151 ISSN: 0305-7453 1460-2091
- LABRECQUE J ET AL: "Effects of a high-molecular-weight cranberry fraction on growth, biofilm formation and adherence of Porphyromonas gingivalis" JOURNAL OF ANTIMICROBIAL CHEMOTHERAPY 200608 GB, vol. 58, no. 2, août 2006 (2006-08), pages 439-443, XP002506152 ISSN: 0305-7453 1460-2091
- STEINBERG D ET AL: "Cranberry high molecular weight constituents promote Streptococcus sobrinus desorption from artificial biofilm" INTERNATIONAL JOURNAL OF ANTIMICROBIAL AGENTS, ELSEVIER SCIENCE, AMSTERDAM, NL, vol. 25, no. 3, 1 mars 2005 (2005-03-01), pages 247-251, XP004763397 ISSN: 0924-8579

## Description

La présente invention concerne un procédé d'obtention d'un extrait préparé à partir du marc de canneberge (genre *Vaccinium macrocarpon et*/*ou Vaccinium oxycoccus*), en -vue d'obtenir un produit qui, présenté sous diverses formes galéniques ou autres, a de nombreuses qualités et propriétés thérapeutiques exploitables notamment dans le domaine des soins et de la prévention dentaire, de l'hygiène buccale, ainsi que dans celui de la prévention des infections virales ou bactériennes de l'oropharynx. Elle concerne également cet extrait et ses utilisations.

Les qualités thérapeutiques de la canneberge sont connues depuis fort longtemps. Attribuées tout d'abord par erreur à son acidité élevée due à la présence d'acides organiques contenus dans le jus, elle a été surtout utilisée pour prévenir et soigner les infections urinaires légères. Il est aujourd'hui connu que ses qualités thérapeutiques sont plutôt dues à des polyphénols contenus dans le jus et la peau de la baie, et plus précisément, à des proanthocyanidines, notamment de type A (*Howell et al. 2005*) qui, selon l'AFSSA, ont la propriété de "contribuer à diminuer la fixation (elles ont donc un effet antiadhésif) de certaines bactéries pathogènes du type E. coli sur les parois des voies urinaires" .

Ce type de préparation a trouvé à ce jour peu d'applications, en raison des inconvénients qu'elle peut présenter dans certains cas ; ainsi, le jus de canneberge, amer et acidulé à la fois (pH 2-3), qui doit être fortement sucré, ne peut être utilisé tel quel pour la prévention ou les soins dentaires puisqu'il peut contribuer à l'érosion amélaire et au développement des caries.

Selon la présente invention, le procédé d'obtention du produit de base susceptible d'être utilisé dans de nombreuses applications n'existe pas dans l'art antérieur.

Les brevets US 5002759 et 5362480 concernent des préparations qui s'opposent à l'adhérence de bactéries pathogènes au niveau des cellules de la paroi buccale ; le genre *Vaccinium* ou la canneberge ne sont pas mentionnés pour qualifier l'extrait.

Le brevet US 5474774 concerne une préparation à base de canneberge, riche en polyphénols, exempte de sucre simple et ayant un effet antiadhésif, mais le procédé d'extraction et la matière première obtenue sont très différents de la présente demande.

Le brevet US 0048611 A1 décrit une préparation à base de canneberge, utilisée pour la fabrication d'un chewing-gum possédant une activité anti-adhésive sur les bactéries . Dans ce cas, la matière première utilisée est un jus de canneberge concentré, et non pas le marc, auquel on fait subir une purification par dialyse . Dans la présente demande, la matière première est un extrait de marc de canneberge totalement déshydraté et la technique par dialyse pour sa préparation n'est pas utilisée.

Le brevet US 4857327 A1 utilise une préparation à base de *Vaccinium* en vue de traiter des troubles digestifs. Les espèces *Vaccinium macrocarpon et Vaccinium oxycoccus* ne sont pas utilisées : seules sont citées *Vaccinium myrtillus, Vaccinium vitis ideae, Sambuccus nigra* et *Vitis vinefera* qui ne possèdent pas de proanthocyanidines de type A, mais uniquement celles de type B. Le marc de canneberge utilisé, exempt de jus, est desséché et incorporé tel quel dans la préparation ; dans la présente demande, il s'agit d'un extrait préparé à partir de ce marc.

Le brevet W099/12541 concerne un extrait de canneberge riche en proanthocyanidines, préparé à partir du fruit, des feuilles, des racines ou tiges de la plante. Le mode d'action physiologique revendiqué mentionne uniquement l'inhibition de l'adhésion des bactéries *E. coli* sur les cellules du tractus urinaire et digestif, alors que la présente demande concerne une action non spécifique à *E. coli.* Les bactéries concernées sont de type *Streptococcus mutans, Porphyromonas gingivalis et Fusobacterium nucleatum* responsables, entre autres, de caries, gingivites, parodontites ; certains virus sont également concernés (Lipson et al 2007).

Aucun de ces brevets ne revendique une activité bactéricide ou bactériostatique.

Le mérite de la présente invention est d'offrir une nouvelle voie de valorisation du marc de canneberge en vue d'obtenir facilement un extrait comportant des proanthocyanidines, notamment de type A, aux propriétés à la fois, antibactériennes, antivirales, antioxydantes et antiadhésives, présenté sous différentes formes galéniques ou autres, utilisées à titre préventif ou curatif, dans les affections dentaires et de l'oropharynx.

Selon un mode de réalisation, le procédé d'obtention fait appel à des techniques qui ne sont pas utilisées dans l'état de la technique antérieure ; il permet d'obtenir un extrait de marc de canneberge, sous forme de poudre, de composition non connue jusqu'alors et plus riche en actifs que ceux que l'on a pu obtenir habituellement.

### OBTENTION DES PROANTHOCYANIDINES A PARTIR DU MARC DE CANNEBERGE

Selon l'invention, la technique proposée pour extraire les proanthocyanidines totales à partir des baies de canneberge comprend plusieurs étapes. La première consiste à obtenir le marc de canneberge par pressurage de la baie de *Vaccinium macrocarpon et*/*ou de Vaccinium oxycoccus,* ce qui permet d'éliminer une grande partie des hydrates de carbone et des acides organiques contenus dans le jus ; la préparation obtenue, d'un pH variant de 3,5 à 4,5 est de ce fait mieux acceptée.

Le marc de canneberge utilisé possède un taux de matière sèche moyen de 30%, ce taux pouvant varier entre 25 et 35% selon la pression exercée durant l'opération de pressurage. Il se présente sous la forme de baies éclatées et les pépins peuvent être conservés puisqu'ils contiennent des proanthocyanidines. Pour des facilités de transport et de stockage, ce marc a été congelé.

Dans un deuxième temps, on procède à l'extraction des proantocyanidines à l'aide d'une solution aqueuse ou à l'aide d'une solution hydro-alcoolique . Le passage des extraits dans une colonne à résine d'adsorption permet de mieux isoler les protoanthocyanidines, donc d'accroître les rendements de l'extraction.

1^{er} cas : l'extraction est réalisée à l'aide d'une solution aqueuse, à température ambiante ou dans une eau préalablement chauffée entre 50 et 70°C. Le marc subit préalablement un broyage afin d'augmenter la surface d'échange solide-liquide, et, de ce fait, le rendement de l'extraction, qui, avec le matériel utilisé et une granulométrie inférieure à 2 mm, augmente de 10 à 20%.

L'extraction par elle même, peut être réalisée sur des proportions de marc de l'ordre de 5 à 20% par rapport à celle de l'eau, la proportion idéale étant de 10%.

Lorsqu'on opère à chaud, dans une eau à 60°C, le marc introduit à raison de 10% dans une cuve munie d'un système d'agitation est soumis à une vitesse d'agitation constante comprise entre 200 et 500 tours/minute selon le type de pâles utilisées. L'extraction peut se poursuivre de 2 à 8 heures, mais l'expérience a montré qu'un temps de 5 heures était le plus approprié afin de ne pas augmenter la proportion de fibres hydrosolubles qui nuisent à la teneur totale de l'extrait en actifs. A l'issue des cinq heures, la suspension subit éventuellement une filtration grossière puis une séparation solide-liquide sur un décanteur à flux horizontaux animé d'une vitesse de rotation variant entre 2500 et 6500 tours/minute ; avec le matériel utilisé, les meilleurs résultats ont été obtenus à 3500 tours/minute. Ce protocole est valable quelle que soit la température de l'eau.

La clarification de la suspension se poursuit ensuite par une centrifugation effectuée à une vitesse comprise entre 4000 et 8000 tours/minute. L'extrait liquide ainsi obtenu subit, lors de la phase suivante, une dessiccation, soit par séchage dans une étuve sous vide à une température inférieure à 60°C afin de ne pas dégrader les actifs, soit par lyophilisation ou bien même par atomisation ou nébulisation afin d'obtenir une phase solide pulvérulente. Des taux d'actifs supérieurs dans le produit final sont obtenus lorsqu'un passage sur résine d'adsorption fait suite à la centrifugation.

Selon l'invention, quelle que soit la méthode employée, le rendement de l'extraction est supérieur à 10% par rapport à la quantité de la matière première sèche de départ. Dans le produit final, le taux de la matière sèche est supérieur à 90%, plutôt compris entre 94 et 97%. Le dosage en proanthocyanidines peut être réalisé par la méthode à la vanilline-acide ou la méthode de Bate Smith bien connues.

Après broyage, la poudre fine et homogène obtenue est de couleur rose à violette.

2^{ème} cas : l'extraction des proanthocyanidiries est effectuée dans ce cas à température ambiante, à l'aide d'une solution hydroalcoolique dont le taux d'éthanol est variable, de 20 à 80%. Les conditions optimales ont été obtenues avec 70% d'éthanol. Les conditions opératoires adoptées sont quasi identiques au cas précédent : le marc de canneberge est introduit à raison de 10% dans le milieu. Le temps d'agitation de la suspension de 3 à 5 heures est également le plus approprié ; cette suspension subit ensuite une décantation dans les mêmes conditions que précédemment. A ce stade, une amélioration de la qualité du produit peut être obtenue par centrifugation puis passage sur résine d'adsorption. Elle permet d'atteindre des taux d'actifs supérieurs dans le produit final, mais, dans ce cas, l'alcool doit être totalement éliminé avant passage de "l'extrait" sur la résine d'adsorption. Le séchage est de même réalisé en étuve sous vide à une température ne dépassant pas 55°C ou bien par lyophilisation. Une variante de ces procédés qui permettent d'obtenir une phase solide pulvérulente, consiste de même à faire subir une atomisation à la suspension.

Le rendement de l'extraction est également, d'environ 10% de poudre sèche par rapport à la matière sèche de départ..

Le taux de matière sèche du produit final est supérieur à 90%, plutôt compris entre 94 et 97%.

Le dosage des proanthocyanidines est effectué également par la méthode à la vanilline-acide ou de Bate-Smith

Après broyage la poudre fine et homogène est de couleur violette.

### Amélioration du rendement en proanthocyanidines par passage sur une résine d'adsorption

Principe : cette opération, lorsqu'elle est bien maîtrisée, permet d'accroître le rendement de l'extraction ; elle consiste à séparer les anthocyanes et les proanthocyanidines des autres substances indésirables contenues dans l'extrait liquide obtenu après épuisement du marc de canneberge par de l'eau à 60°C ou par la solution hydroalcoolique. Après avoir subi une clarification par filtration grossière, décantation puis par centrifugation, les polyphénols contenus dans l'extrait liquide après élimination de l'alcool s'il en contient, vont s'adsorber sur la résine et le restant est éliminé. Il suffit ensuite de faire une élution pour récupérer les actifs.

Les colonnes d'absorption utilisées sont en acier inoxydable type 316L, remplies de résine adsorbante de type silices greffées FPX 66, ou de toute autre résine aux propriétés équivalentes. Les résines sont activées grâce à un bain d'éthanol à 96% pendant 24 heures au minimum. Elles sont ensuite rincées à l'eau jusqu'à l'élution totale de l'alcool.

Séparation des actifs : la colonne préparée dans ce but contient un volume BV (Bed volume) de résine adsorbante. Un volume BV de l'extrait liquide de canneberge est chargé, à contre courant, par le bas de la colonne, à une vitesse de 2BV/heure. L'eau dans laquelle baigne la résine est éliminée par le haut ; de même, la fraction non fixée, éluée directement est récupérée.

Lorsque le volume BV de l'extrait liquide a été chargé, un rinçage à l'eau est effectué cette fois, par le haut de la colonne à une vitesse de 2 BV/heure . Il a pour but d'éluer les composés non adsorbés sur les résines, notamment les substances non phénoliques et les sucres ; ce rinçage est poursuivi jusqu'à ce que le taux de matière sèche de l'éluat soit inférieur à 0,5%, ce qui correspond approximativement à un volume 3 BV d'eau.

La récupération des polyphénols adsorbés sur les résines est effectuée ensuite avec de l'éthanol à 75% introduit par le haut de la colonne à raison de 2 BV/heure. La récupération des composés polyphénoliques est poursuivie jusqu'à la disparition totale de la couleur violette de l'éluat.

Le volume généralement nécessaire pour éluer la totalité des polyphénols adsorbés sur la résine est de l'ordre de 2 BV. A ce stade, un dosage des polyphénols est souhaitable pour s'assurer que la totalité des polyphénols, qui sont pour le moins de l'ordre de 20% de la quantité de matière sèche de départ, a été éluée.

L'éluat alcoolique récupéré est alors concentré sous vide partiel, à une température inférieure à 55°C ; simultanément, une addition régulière d'eau dans ce milieu hydroalcoolique prévient tout risque de détérioration des polyphénols. Au stade terminal, les proanthocyanidines se trouvent dans un milieu 100% aqueux. Comme déjà mentionné, le produit subit ensuite une dessiccation dans une étuve sous vide partiel ou une lyophilisation, de préférence ; il peut de même subir une atomisation.

La poudre fine, violette à noire, contient à ce stade au moins 20% de proanthocyanidines totales.

Selon la méthode d'extraction et la méthode de dosage utilisées, les taux de proanthocyanidines totales (PACs) obtenus dans l'extrait sec final, autrement dit, les taux de pureté moyen sont les suivants avec 1a :
- Méthode à la vanilline-acide :
   o 1 à 10% de PACs sur matière sèche pour un extrait aqueux
   o 10 à 20% de PACs sur matière sèche pour un extrait alcoolique
   o supérieur à 20% de PACs sur matière sèche pour un extrait après passage sur résine.
- Méthode de Bate Smith :
   o 5 à 20% de PACs sur matière sèche pour un extrait aqueux
   o 10 à 30% de PACs sur matière sèche pour un extrait alcoolique
   o supérieur à 40% et même plus de 50% de PACs sur matière sèche pour un extrait après passage sur résine.

### APPLICATIONS

L'extrait de marc de canneberge, du fait principalement de sa richesse en proanthocyanidines notamment de type A, solubles à différents pH (et donc dans la salive) et aux propriétés antibactériennes, antivirales, antioxydantes, antiadhésives, peut être utilisé, à différentes concentrations et sous des formes galéniques ou autres variées, à titre préventif ou curatif, pour lutter contre les affections dentaires ou de l'oropharynx chez l'homme et l'animal.

Le produit final peut être présenté finement broyé, granulé ou encapsulé notamment sous forme de comprimés, comprimés orodispersibles, de gélules, de pastilles à base de gomme arabique. Etant donné sa compatibilité avec la plupart des excipients utilisés dans le domaine pharmaceutique, ce type de préparation peut notamment, sans problème, être aromatisé par des arômes fruités ou des huiles essentielles, édulcoré par de l'aspartame, de l'acésulfame, du stevia rebaudiana, coloré par des colorants alimentaires autorisés. De même, comme il est également compatible avec des gélifiants tels les pectines, les gommes (xanthane, gomme arabique, gellane), les dextrines, les émulsifiants, les polysaccharides, les glucomannanes, les polyols comme le sorbitol, le xylitol, le maltilol, le lactilol, il peut être inclus, par exemple, dans des tablettes de chewing gum à base de polyols et de glycérine, incorporé dans des dentifrices sous forme de pâtes ou de gels à base de résines carboxyvinyliques, des bains de bouche par dissolution dans de l'eau glycérinée et d'une manière générale, dans toute forme solide, liquide, ou pâteuse, administrée par voie orale et permettant sa libération et dissolution au niveau buccal et de l'oropharynx.

L'efficacité de ces proanthocyanidines sur les bactéries buccales a été prouvée par des tests antibactériens et des tests d'adhérence réalisés avec trois types d'extraits de canneberge dont les concentrations sont respectivement de 2 mg/ml, 5 mg/ml et 10 mg/ml, dénommés pour l'expérimentation Exocyan cran 1, Exocyan cran 2 et Exocyan cran 10 ; chacun de ces extraits a été préparé à partir d'extraits secs en proanthocyanes titrés à 1%, 2% et 10%, ce qui veut dire par exemple, que la série exocyan cran 1 préparée à partir d'extraits secs à 1% a permis d'obtenir les trois types d'extraits aux concentrations respectives de 2 mg/ml, 5 mg/ml et 10 mg/ml.

### Tests antibactériens :

L'efficacité des extraits de canneberge peut être évaluée in vitro. Les produits sont considérés comme ayant un caractère bactériostatiques lorsque le nombre de germes diminue pour le moins de 1/10 (un log vs témoin ), ou sont considérés comme bactéricides lorsqu'il y a inhibition totale de la culture bactérienne.

Si les extraits sont considérés comme actifs in vitro par rapport à un milieu témoin reproduisant le milieu salivaire, il faut s'attendre à ce qu'ils aient la même activité in vivo ; la salive, en assurant la dispersion des proanthocyanidines dans la cavité buccale, la gorge et le système digestif, permet à l'actif de se trouver au contact des cellules des muqueuses et d'inhiber la formation du biofilm.

Résultats : il a été constaté que les trois extraits à la concentration de 10 mg/ml ont une activité bactériostatique sur *Streptoccus mutans*, ce qui permet de penser qu'ils ont une activité bactéricide à une concentration supérieure (effet dose dépendant).

Seul l'extrait Exocyan cran 10, à la concentration de 10mg/ml, a une activité bactériostatique sur *Porphyromonas gingivalis.*

*Fusobactérium nucleatum* est la bactérie la plus sensible aux trois extraits à la concentration de 10 mg/ml ; Exocyan cran 1 et Exocyan cran 10 ont une activité bactéricide tandis que Exocyan cran 2 montre une activité bactériostatique.

La croissance de *Lactobacillus rhamnosus* n'est modifiée par aucun des extraits, et ceci quelle que soit leur concentration.

### Contrôle de l'effet antiadhésif :

Les bactéries ont la propriété d'adhérer les unes aux autres pour former un biofilm sous lequel prolifèrent les microorganismes responsables des caries, de la gingivite et des maladies parodontales.

On considère que l'on a un résultat positif lorsqu'il se produit une inhibition totale ou partielle du biofilm (scores de 0 à 3), l'inhibition la plus faible étant obtenue avec le score le plus élevé.

Il a été observé que les trois extraits aux concentrations de 5 mg/ml ont une action sur le biofilm de *Streptococcus mutans* ; il en est de même pour Exocyan cran 1 et 10 à la concentration de 10 mg/ml (score =1) ; toutefois, Exocyan cran 1 a montré une activité inhibitrice plus faible (score = 3) que les autres extraits.

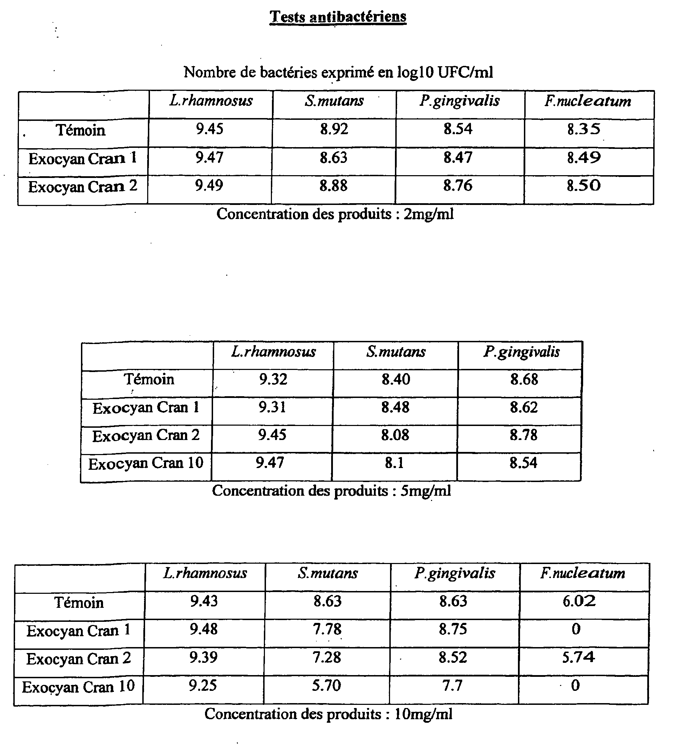

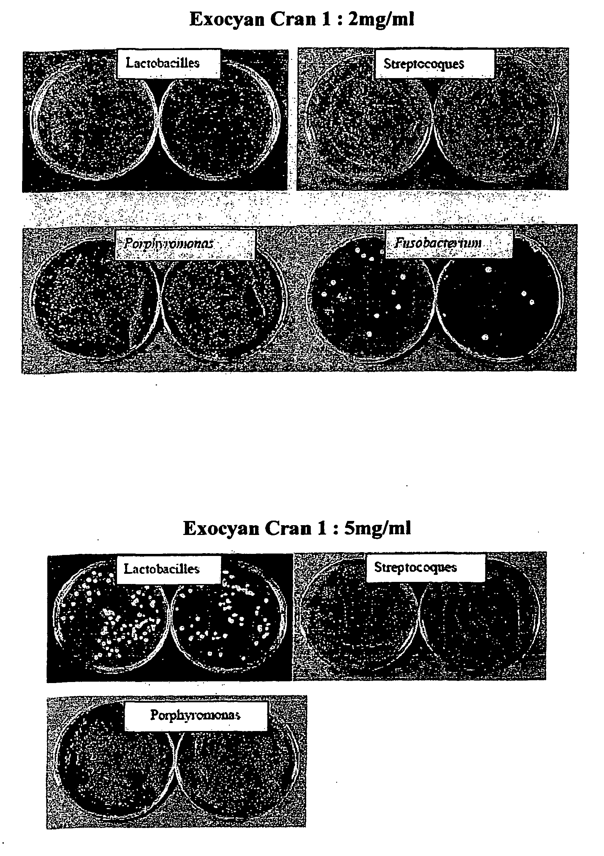

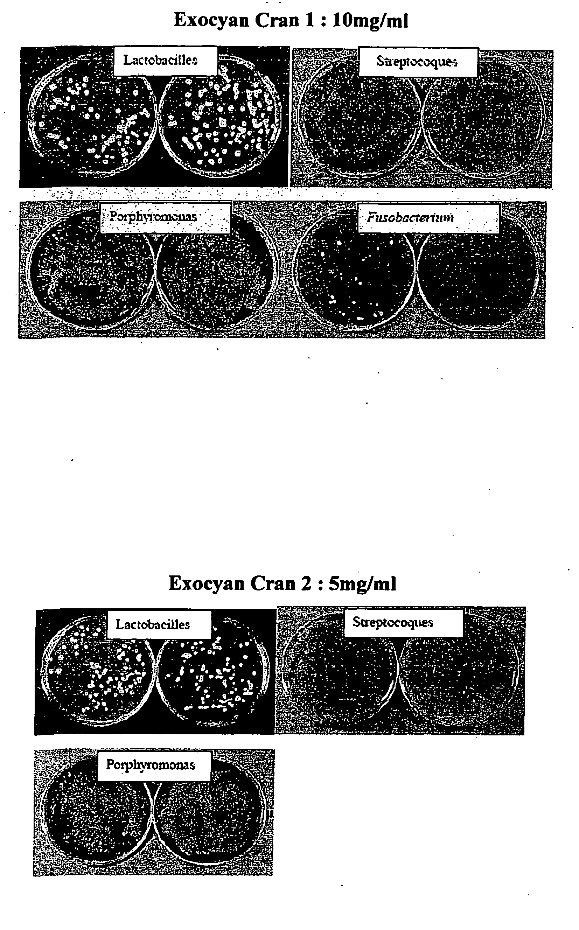

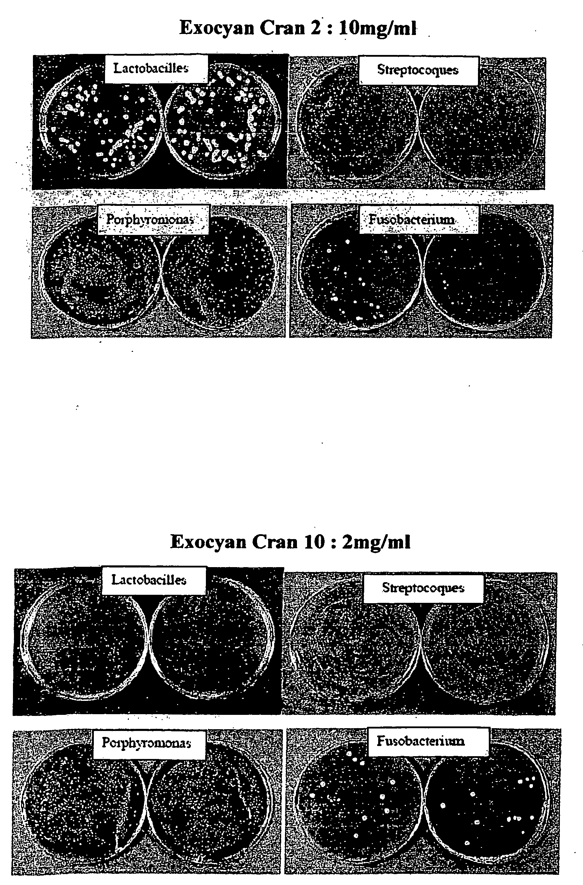

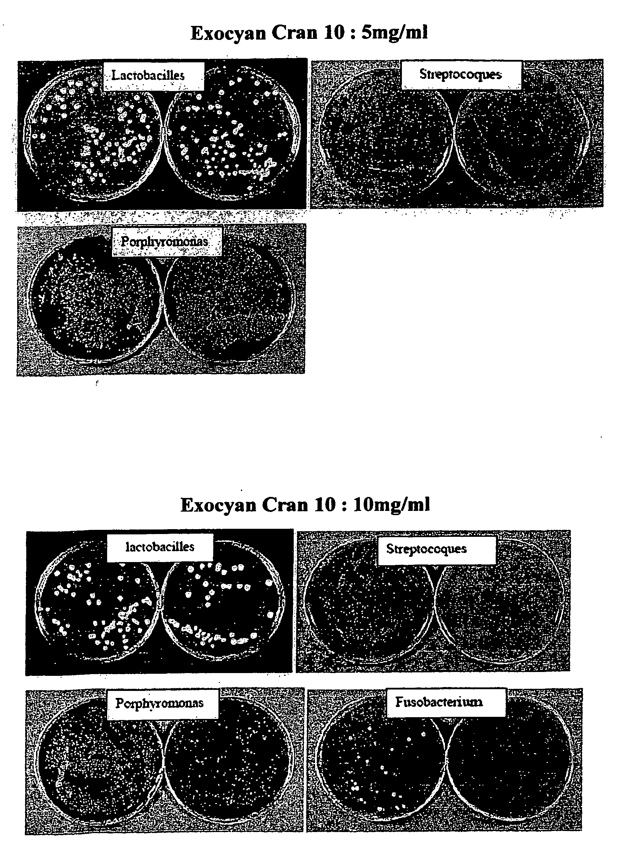

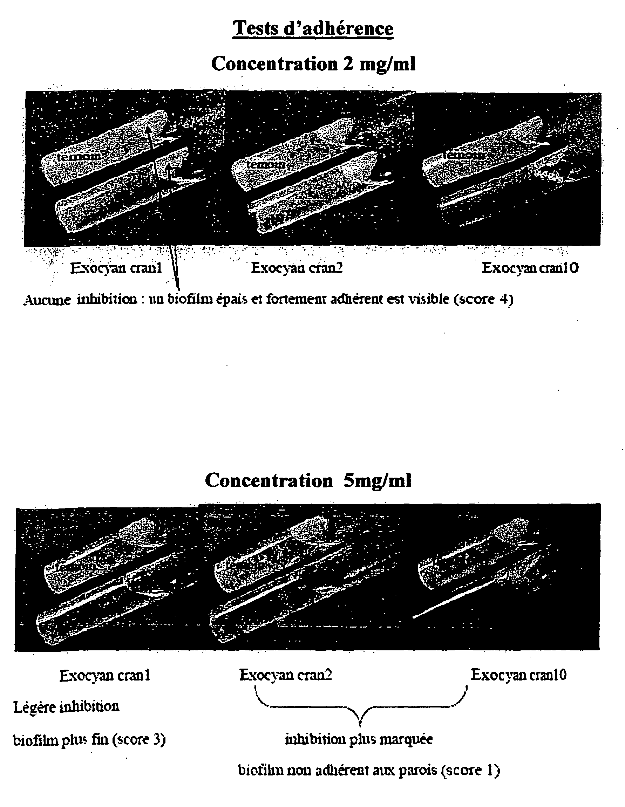

## Revendications

1. Procédé d'obtention d'un extrait de canneberge **caractérisé en ce qu'**un extrait de marc de canneberge, comportant un fort pourcentage de proanthocyanidines de type A compns, entre 1% et plus de 20% avec la méthode à la vanilline-acide et entre 5% et plus de 50% avec la méthode de Bate Smith, présentant des propriétés antibactériennes, antivirales et antiadhésives, est obtenu à partir de la baie de canneberge, genre *Vaccinium macrocarpon* et/ou *Vaccinium oxycoccus :*
- par pressurage de la baie,
- puis extraction des proanthocyanidines contenues dans le marc ainsi obtenu à l'aide d'une solution aqueuse à chaud ou à température ambiante ou d'une solution hydro-alcoolique à température ambiante, opération qui est suivie par des techniques de séparation solide-liquide connues,
- puis d'un passage sur résine d'adsorption de type silices greffées permettant d'isoler les anthocyanes et les proanthocyanidines des substances indésirables et de
- récupérer à partir de l'éluat ces mêmes proanthocyanidines sous forme de poudre sèche par séchage sous vide, ou lyophilisation, ou atomisation ou nébulisation.

2. Procédé d'obtention d'un extrait de canneberge, selon la revendication 1 **caractérisé en ce que** l'extrait de marc de canneberge est obtenu par extraction aqueuse des proanthocyanidines à partir du marc de canneberge broyé, puis mélangé dans une proportion de 5 à 20% à de l'eau à température ambiante ou portée à une température comprise entre 50 et 70°C, le tout maintenu sous agitation à une vitesse constante comprise entre 200 et 500 tours/minute pendant 2 à 8 heures, les matières hydrosolubles qui contiennent les proanthocyanidines étant ensuite séparées des matières insolubles par filtration grossière, passage sur un décanteur à flux horizontaux dont la vitesse de rotation est comprise entre 2500 et 6500 tours/minute, centrifugation, puis isolées par passage sur une résine d'adsorption de type silices greffées, récupérées et séchées par séchage sous vide, ou lyophilisation, ou atomisation ou nébulisation, afin d'obtenir une phase solide pulvérulente.

3. Procédé d'obtention d'un extrait de canneberge, selon la revendication 1, **caractérisé en ce que** l'extrait de marc de canneberge est obtenu par extraction hydro-alcoolique des proanthocyanidines à partir du marc de canneberge broyé puis mélangé, à raison de 5 à 20% à la solution aqueuse contenant 20 à 80% d'éthanol, le tout maintenu sous agitation à une vitesse constante comprise entre 200 à 500 tours/minute, à température ambiante, durant 2 à 8 heures, les matières solubles qui contiennent les proanthocyanidines étant alors séparées des matières insolubles par filtration grossière, passage sur un décanteur à flux horizontaux dont la vitesse de rotation est comprise entre 2500 et 6500 tours/minute, centrifugation, puis isolées par passage sur une résine d'adsorption de type silices greffées après élimination de l'alcool, récupérées et séchées par séchage sous vide, lyophilisation, atomisation ou nébulisation, afin d'obtenir une phase solide pulvérulente.

4. Procédé d'obtention d'un extrait de canneberge selon la revendication 2, **caractérisé en ce que** l'extrait de canneberge obtenu par extraction aqueuse des proanthocyanidines à partir du marc de canneberge broyé, est mélangé dans une proportion de 10% à de l'eau à température ambiante ou portée à une température de 60°C, le tout étant maintenu sous agitation à une vitesse constante comprise entre 200 et 500 tours/minute pendant une durée de cinq heures, les matières hydrosolubles qui contiennent les proanthocyanidines étant ensuite séparées des matières insolubles par filtration grossière, passage sur un décanteur à flux horizontaux dont la vitesse de rotation est de 3500 tours/minute, centrifugation, puis isolées par passage sur une résine d'adsorption de type silices greffées, récupérées et séchées par séchage sous vide, ou lyophilisation, ou atomisation ou nébulisation, afin d'obtenir une phase solide pulvérulente.

5. Procédé d'obtention d'un extrait de canneberge selon la revendication 3, **caractérisé en ce que** l'extrait de canneberge est obtenu par extraction hydro-alcoolique des proanthocyanidines à partir du marc de canneberge broyé puis mélangé dans la proportion de 10% à la solution aqueuse contenant 70% d'éthanol, le tout maintenu sous agitation à une vitesse constante comprise entre 200 à 500 tours/minute, à température ambiante, pendant une durée de 5 heures, les matières solubles qui contiennent les proanthocyanidines étant alors séparées des matières insolubles par filtration grossière, passage sur un décanteur à flux horizontaux dont la vitesse de rotation est de 3500 tours/minute, centrifugation, puis isolées par passage sur une résine d'adsorption de type silices greffées après élimination de l'alcool, récupérées et séchées par séchage sous vide, lyophilisation, atomisation ou nébulisation, afin d'obtenir une phase solide pulvérulente.

6. Procédé selon l'une quelconque des revendications précédentes, **caractérisé en ce que**, après passage sur la résine d'adsorption de type silices greffées, le rendement de l'extraction obtenue au moyen d'une solution aqueuse ou au moyen d'une solution hydro-alcoolique est supérieur à 10% par rapport à la quantité de matière sèche de départ, et le taux de matière sèche dans le produit final est supérieur à 90%.

7. Procédé selon la revendication 6, **caractérisé en ce que** le taux de matière sèche dans le produit final est compris entre 94 et 97%.

8. Extrait de canneberge **caractérisé en ce que**, obtenu à partir du marc de canneberge selon les revendications 1, 2 ou 3, comportant des proanthocyanidines de type A, ayant des propriétés antibactériennes, antivirales et antiadhésives, pour être utilisé, associé à des excipients pharmaceutiques et sous des formes galéniques ou autres telles que pastilles, tablettes de chewing-gum, dentifrices, bains de bouche, à titre préventif ou curatif, pour le traitement des affections dentaires ou de l'oropharynx, chez l'homme et l'animal.

## Claims

1. A method for obtaining an extract of cranberry **characterised in that** an extract of cranberry pomace, containing a high percentage of type A proanthocyanidins of between 1% and more than 20 % with the vanillin-acid method and between 5 % and more than 50 % with the Bate Smith method, having antibacterial, antiviral and antiadhesive properties, is obtained from the cranberry berry, genus *Vaccinium macrocarpon* and/or *Vaccinium oxycoccus*:
- by pressing the berry,
- then by extracting the proanthocyanidins contained within the pomace thus obtained using an aqueous solution either heated or at room temperature or a hydroalcoholic solution at room temperature, this operation being followed by known solid-liquid separation techniques,
- then by passing through an adsorption resin such as a grafted silica resin enabling the anthocyanins and proanthocyanidins to be isolated from the unwanted substances and by
- recovering from the eluate these same proanthocyanidins in the form of a dry powder via vacuum drying, freeze drying, atomisation or nebulisation.

2. A method for obtaining an extract of cranberry according to claim 1, **characterised in that** the cranberry pomace extract is obtained by the aqueous extraction of proanthocyanidins from the cranberry marc which is ground, then mixed at the proportion of 5 to 20 % in water at room temperature or brought to a temperature of between 50 and 70°C, while permanently being mixed at a constant speed of between 200 and 500 revolutions/minute for 2 to 8 hours, the water-soluble material containing the proanthocyanidins then being separated from the insoluble material by coarse filtration, passing through a decanter with horizontal flow, the rotation speed of which is between 2500 and 6500 revolutions/minute, centrifugation, then isolated by passing through an adsorption resin such as a grafted silica resin, recovered and dried by vacuum drying, freeze drying, atomisation or nebulisation, in order to obtain a powdery solid phase.

3. A method for obtaining an extract of cranberry according to claim 1, **characterised in that** the cranberry pomace extract is obtained by the hydroalcoholic extraction of proanthocyanidins from the cranberry pomace which is ground,then mixed at the proportion of 5 to 20 % in the aqueous solution containing 20 to 80 % ethanol, while permanently being mixed at a constant speed of between 200 to 500 revolutions/minute, at room temperature, for 2 to 8 hours, the soluble material containing the proanthocyanidins thus being separated from the insoluble material by coarse filtration, passing through a decanter with horizontal flow, the rotation speed of which is between 2500 and 6500 revolutions/minute, centrifugation, then isolated by passing through an adsorption resin such as a grafted silica resin after removing the alcohol, recovered and dried by vacuum drying, freeze drying, atomisation or nebulisation, in order to obtain a powdery solid phase.

4. A method for obtaining an extract of cranberry according to claim 2, **characterised in that** the cranberry extract obtained by the aqueous extraction of proanthocyanidins from the cranberry pomace which is ground, is mixed at a proportion of 10 % in water at room temperature or brought to a temperature of 60°C, while permanently being mixed at a constant speed of between 200 and 500 revolutions/minute for five hours, the water-soluble material containing the proanthocyanidins then being separated from the insoluble material by coarse filtration, passing through a decanter with horizontal flow, the rotation speed of which is 3500 revolutions/minute, centrifugation, then isolated by passing through an adsorption resin such as a grafted silica resin, recovered and dried by vacuum drying, freeze drying, atomisation or nebulisation, in order to obtain a powdery solid phase.

5. A method for obtaining an extract of cranberry according to claim 3, **characterised in that** the cranberry extract is obtained by the hydroalcoholic extraction of proanthocyanidins from the cranberry pomace which is ground, then mixed at the proportion of 10 % in the aqueous solution containing 70 % ethanol, while permanently being mixed at a constant speed of between 200 to 500 revolutions/minute, at room temperature, for 5 hours, the soluble material containing the proanthocyanidins thus being separated from the insoluble material by coarse filtration, passing through a decanter with horizontal flow, the rotation speed of which is 3500 revolutions/minute, centrifugation, then isolated by passing through an adsorption resin such as a grafted silica resin after removing the alcohol, recovered and dried by vacuum drying, freeze drying, atomisation or nebulisation, in order to obtain a powdery solid phase.

6. A method according to any one of the previous claims, **characterised in that**, after passing through the adsorption resin such as a grafted silica resin, the quantity of extract obtained by means of an aqueous solution or by means of a hydroalcoholic solution is more than 10 % when compared to the quantity of initial dry material, and the quantity of dry material in the end product is more than 90 %.

7. A method according to claim 6, **characterised in that** the quantity of dry material in the end product is between 94 and 97 %.

8. An extract of cranberry **characterised in that**, obtained from the cranberry pomace according to claims 1, 2 or 3, containing type A proanthocyanidins, having antibacterial, antiviral and antiadhesive properties, to be used, associated with pharmaceutical excipients and in galenic forms or other forms such as lozenges, chewing-gum tablets, toothpastes, mouthwashes, for preventive or curative purposes, for treating dental or oropharynx-related conditions in humans and animals.

## Patentansprüche

1. Verfahren zum Erhalt eines Moosbeerextraktes, **dadurch gekennzeichnet, dass** man aus der Moosbeere, *Vaccinium macrocarpon* und/ oder *Vaccinium oxycoccus* ein Moosbeermaische-Extrakt mit einem hohen Prozentsatz an A-Typ-Proanthocyanidinen, zwischen 1% und mehr als 20% nach der Vanillinsäure-Methode und zwischen 5% und mehr als 50% nach der Bate-Smith-Methode, mit antibakteriellen, antiviralen und antiadhäsiven Eigenschaften erhält:
- durch Auspressen der Beere,
- danach durch das Extrahieren der Proanthocyanidine, die in der Maische enthalten sind, die man mithilfe einer erhitzten oder auf Umgebungstemperatur befindlichen wässrigen Lösung, oder mithilfe einer auf Umgebungstemperatur befindlichen hydroalkoholischen Lösung erhält, eines Vorganges, dem bekannte Techniken zum Trennen von Feststoffen und Flüssigkeiten folgen,
- danach durch einen Durchlauf auf einem Adsorberharz, wie beispielsweise einer modifizierten Kieselsäure, zum Trennen der Anthocyane und der Proanthocyanidine und unerwünschten Begleitsubstanzen, und
- durch die Gewinnung derselben Proanthocyanidine in Form eines Trockenpulvers aus dem Eluat durch Vakuumtrocknung, oder Gefriertrocknung oder Atomisierung oder Vernebelung.

2. Verfahren zum Erhalt eines Moosbeerextraktes nach Anspruch 1, **dadurch gekennzeichnet, dass** das Moosbeermaische-Extrakt durch die wässrige Extraktion der Proanthocyanidine aus der zerstoßenen Moosbeermaische erhalten wird, und danach in einem Verhältnis von 5% bis 20% mit Wasser auf Umgebungstemperatur oder mit Wasser vermischt wird, das auf eine Temperatur zwischen 50 und 70°C erhitzt wird, wobei alles zusammen 2 bis 8 Stunden lang mit einer konstanten Geschwindigkeit zwischen 200 und 500 Umdrehungen pro Minute gerührt wird, und die wasserlöslichen Stoffe, die die Proanthocyanidine enthalten, durch eine grobe Filterung, den Durchlauf durch einen Horizontalstrom-Abscheider, dessen Rotationsgeschwindigkeit zwischen 2500 und 6500 Umdrehungen/ Minute liegt, und Zentrifugierung von den unlöslichen Stoffen getrennt, danach durch einen Durchlauf auf einem Adsorberharz, wie beispielsweise einer modifizierten Kieselsäure isoliert, durch Vakuumtrocknung oder Gefriertrocknung oder Atomisierung oder Vernebelung aufgefangen und getrocknet werden, um dann eine feste pulvrige Phase zu ergeben.

3. Verfahren zum Erhalt eines Moosbeerextraktes nach Anspruch 1, **dadurch gekennzeichnet, dass** das Moosbeermaische-Extrakt durch eine hydroalkoholische Extraktion der Proanthocyanidine aus der zerstoßenen Moosbeermaische erhalten wird, und danach in einem Verhältnis von 5% bis 20% mit der wässrigen Lösung vermischt wird, die 20 bis 80% Ethanol enthält, wobei alles zusammen 2 bis 8 Stunden lang mit einer konstanten Geschwindigkeit zwischen 200 und 500 Umdrehungen pro Minute bei Umgebungstemperatur gerührt wird, und die wasserlöslichen Stoffe, die die Proanthocyanidine enthalten, durch eine grobe Filterung, den Durchlauf durch einen Horizontalstrom-Abscheider, dessen Rotationsgeschwindigkeit zwischen 2500 und 6500 Umdrehungen/ Minute liegt, und Zentrifugierung von den unlöslichen Stoffen getrennt, danach, und nach Ausscheidung des Alkohols, durch einen Durchlauf auf einem Adsorberharz, wie beispielsweise einer modifizierten Kieselsäure isoliert, durch Vakuumtrocknung oder Gefriertrocknung oder Atomisierung oder Vernebelung aufgefangen und getrocknet werden, um dann eine feste pulvrige Phase zu ergeben.

4. Verfahren zum Erhalt eines Moosbeerextraktes nach Anspruch 2, **dadurch gekennzeichnet, dass** der durch eine wässrige Extraktion der Proanthocyanidine aus der zerstoßenen Moosbeermaische erhaltene Moosbeerextrakt in einem Verhältnis von 10% mit Wasser auf Umgebungstemperatur oder mit Wasser vermischt wird, das auf eine Temperatur von 60°C erhitzt wird, wobei alles zusammen 5 Stunden lang mit einer konstanten Geschwindigkeit zwischen 200 und 500 Umdrehungen pro Minute gerührt wird, und die wasserlöslichen Stoffe, die die Proanthocyanidine enthalten, durch eine grobe Filterung, den Durchlauf durch einen Horizontalstrom-Abscheider, dessen Rotationsgeschwindigkeit bei 3500 Umdrehungen/ Minute liegt, und Zentrifugierung von den unlöslichen Stoffen getrennt, und dann durch einen Durchlauf auf einem Adsorberharz, wie beispielsweise einer modifizierten Kieselsäure isoliert, durch Vakuumtrocknung oder Gefriertrocknung oder Atomisierung oder Vernebelung aufgefangen und getrocknet werden, um dann eine feste pulvrige Phase zu ergeben.

5. Verfahren zum Erhalt eines Moosbeerextraktes nach Anspruch 3, **dadurch gekennzeichnet, dass** der Moosbeerextrakt durch die hydroalkoholische Extraktion der Proanthocyanidine aus der zerstoßenen Moosbeermaische erhalten wird, und danach in einem Verhältnis von 10% mit der wässrigen Lösung vermischt wird, die 70% Ethanol enthält, wobei alles zusammen 5 Stunden lang mit einer konstanten Geschwindigkeit zwischen 200 und 500 Umdrehungen pro Minute bei Umgebungstemperatur gerührt wird, und die wasserlöslichen Stoffe, die die Proanthocyanidine enthalten, durch eine grobe Filterung, den Durchlauf durch einen Horizontalstrom-Abscheider, dessen Rotationsgeschwindigkeit bei 3500 Umdrehungen/ Minute liegt, und Zentrifugierung von den unlöslichen Stoffen getrennt, danach, und nach Ausscheidung des Alkohols, durch einen Durchlauf auf einem Adsorberharz, wie beispielsweise einer modifizierten Kieselsäure isoliert, durch Vakuumtrocknung oder Gefriertrocknung oder Atomisierung oder Vernebelung aufgefangen und getrocknet werden, um dann eine feste pulvrige Phase zu ergeben.

6. Verfahren nach irgendeinem der vorherigen Ansprüche, **dadurch gekennzeichnet, dass** die Extraktionsrate, die man durch eine wässrige oder eine hydroalkoholische Lösung nach erfolgtem Durchlauf auf einem Adsorberharz, wie beispielsweise einer modifizierte Kieselsäuren erhält, im Verhältnis zur ursprünglichen Menge an Trockensubstanz bei über 10% liegt, und der Trockensubstanzanteil im Endprodukt bei über 90% liegt.

7. Verfahren nach Anspruch 6, **dadurch gekennzeichnet, dass** der Trockensubstanzanteil im Endprodukt zwischen 94 und 97% liegt.

8. Moosbeerextrakt, **dadurch gekennzeichnet, dass** er nach den Ansprüchen 1, 2 oder 3 aus der Moosbeermaische gewonnen wird, und A-Typ-Proanthocyanidine mit antibakteriellen, antiviralen und antiadhäsiven Eigenschaften umfasst, um in vorbeugender oder heilender Form, und gemeinsam mit pharmazeutischen Grundmassen, sowie in galenischen oder anderen Formen, wie Lutschtabletten, Kaugummitabletten, Zahnpasten, Mundduschen, zur Behandlung von Zahnschmerzen und Schmerzen im Mund-Rachenraum bei Mensch und Tier angewandt zu werden.
